# EUROPEAN PATENT SPECIFICATION

(11) **EP 1 287 827 B1**
(45) Date of publication and mention of the grant of the patent: **29.12.2004**
(21) Application number: 01930134.0
(22) Date of filing: 15.05.2001
(51) Int. Cl.: A61K 31/715, A61K 35/78, A61P 37/04, C08B 37/14

(54) **IMMUNOPOTENTIATOR**
IMMUNOAKTIVATOR
IMMUNOPOTENTIALISATEUR

(30) Priority: 15.05.2000 JP 2000141181
(43) Date of publication of application: 05.03.2003
(73) Proprietor: Nihon Shokuhin Kako Co., Ltd., Shibuya-ku, Tokyo 151-0051 (JP)
(72) Inventor: TAKEUCHI, Masayasu, Fuji-shi Shizuoka-ken 417-0001 (JP); NAKAMURA, Nobuyuki, Mishima-shi Shizuoka-ken 411-0802 (JP)
(74) Representative: Beacham, Annabel Rose
(86) International application number: PCT/JP2001/004017
(87) International publication number: WO 2001/087311

(56) References cited:
- EP-A- 0 457 539
- US-A- 5 622 738
- PATENT ABSTRACTS OF JAPAN vol. 1996, no. 04, 30 April 1996 (1996-04-30) & JP 07 324036 A (NIPPON SHOKUHIN KAKO CO LTD;OTHERS: 02), 12 December 1995 (1995-12-12)
- DATABASE WPI Section Ch, Week 199216 Derwent Publications Ltd., London, GB; Class D13, AN 1992-128207 XP002231702 & JP 04 071466 A (NIPPON SHOKUHIN KAKO KK) , 6 March 1992 (1992-03-06)
- PATENT ABSTRACTS OF JAPAN vol. 012, no. 056 (C-477), 19 February 1988 (1988-02-19) & JP 62 201821 A (NIPPON SHOKUHIN KAKO KK), 5 September 1987 (1987-09-05)
- DATABASE WPI Section Ch, Week 199309 Derwent Publications Ltd., London, GB; Class A11, AN 1993-071128 XP002231703 & JP 05 017503 A (TERUMO CORP), 26 January 1993 (1993-01-26)
- PATENT ABSTRACTS OF JAPAN vol. 1999, no. 06, 31 March 1999 (1999-03-31) & JP 06 316525 A (ALCON LAB INC), 15 November 1994 (1994-11-15)

## Description

### Technical Field

The present invention relates to an immuno-active agent obtained from cornhusk as a starting material.

### Background Art

Immune system is one of important protective mechanisms of an organism to protect the organism from invasion of extraneous viruses or microorganisms, eliminate foreign matters such as cancer cells and maintain the homeostasis of the organism. It is believed that if the immunological function deteriorates due to elements such as aging, stress, fatigue or environmental factors, various diseases or infectious illnesses are easily caused.

Accordingly, it is important to strengthen the immunological power as the protective mechanism of the organism for the purpose of maintaining the homeostasis of the organism, and immuno-active substances which activate the immunological mechanism can be widely used for foods and medicines.

As immuno-active substances or methods for the production thereof, various ones have been proposed, and as one of them, partially hydrolyzed products of hemicellulose of rice bran have been known.

For example, Japanese Patent No. 2532899 (JP-A-1-153701) discloses a method for producing hemicellulose having immuno-active action which is obtained by extracting hemicellulose from rice bran with an alkali and hydrolyzing the hemicellulose with filamentous fungi. JP-A-9-23895 discloses a method for forming immunity-potentiating substances by preparing water-soluble polysaccharides from plant tissues and hydrolyzing them with exoenzymes of filamentous fungi.

However, with respect to the immuno-active effects of the immunity-potentiating substances or the hemicellulose derived from the rice bran, which are obtainable by the above production methods, disclosed are only the increase of natural killer cells (NK-cells) in vitro and the measurements of their activating effects. Accordingly, it has been unclear whether or not the immuno-active action can be seen even when these are actually orally administered.

Further, particularly in the former production method, it is difficult to obtain reaction products having a constant quality and further, it takes as long as 15 days for cultivation of filamentous fungi. Accordingly, such a method can not be called a preferred method for production on an industrial scale.

Moreover, it has been unknown about whether or not hemicellulose prepared from cornhusk and its partially hydrolyzed product have immuno-active action.

### Disclosure of Invention

Accordingly, it is an object of the present invention to provide an immuno-active agent derived from cornhusk which has an action for activating the immunological power of organism by oral administration.

The present inventors have conducted intensive studies for the purpose of achieving the above object, and as a result, found that partially hydrolyzed hemicellulose prepared from cornhusk has immuno-active action. They have accomplished the present invention based on this discovery.

Namely, the present invention provides use of a partially hydrolyzed hemicellulose obtained from cornhusk and hydrolyzed with xylanase, wherein the hemicellulose is a hemicellulose obtained by removing starch and protein from the cornhusk and subjecting a residue to extraction with alkali at a temperature of 80 to 140°C, in the manufacture of a composition for use in enhancing an immune response.

According to the present invention, it is possible to provide an immuno-active agent which is safe and can activate the immunological power by oral administration.

### Best Mode for Carrying out the Invention

The method for preparing the partially hydrolyzed hemicellulose obtained from cornhusk as the active ingredient of the immuno-active agent, is not particularly limited, and conventional methods may be applied.

For example, as the method for extracting hemicellulose from cornhusk, extraction with alkalis or acids, extraction at high pressure and high temperature with an extruder or an autoclave, extraction using enzymes such as cellulases, or a method having these methods appropriately combined, may be used. In the present invention, in order to obtain hemicellulose with a high purity, starch and protein, and if the case requires, lipids, inorganic substances and the like, are removed from the cornhusk, and the residues subjected to alkali extraction to prepare hemicellulose.

As the method for removing starch and protein, and if the case requires, lipids, inorganic substances and the like, from the cornhusk, any one of enzymatic treatments, chemical treatments and physical treatments may be used, or these treatments may be appropriately combined. The enzymatic treatments are made by adding, for example, a starch-hydrolyzing enzyme such as α-amylase or glucoamylase, a protein-hydrolyzing enzyme such as protease, a lipid-hydrolyzing enzyme such as lipase, or a cellulose-hydrolyzing enzyme such as cellulase, under the conditions of pH 3 to 9 and a temperature of from 30 to 100 °C, to let the enzyme act for the treatment. Further, the chemical treatments are made by adding, for example, an aqueous solution of a mineral acid or an organic acid to the cornhusk, followed by heating under the condition of pH 2 to 5, or adding a surface active agent for food, and subjecting it to heat treatment under the condition of pH 3 to 8. Moreover, the physical treatments are made by, for example, pulverizing the cornhusk with a homogenizer, a hammer mill or the like, followed by sorting.

Further, as the method for partially hydrolyzing the hemicellulose, a xylanase hemi-cellulose-hydrolyzing enzyme is used.

The hemicellulose used in the present invention may be prepared, for example, as mentioned below.

80 to 95 parts by mass of water is added to 5 to 20 parts by mass of the cornhusk preferably pre-treated as above, an alkali compound is added thereto to adjust the pH to 10 to 13, and this is stirred and mixed at 80 to 140 °C for 0.5 to 10 hours, to extract hemicellulose. As the alkali compound, there is no particular limitation, and calcium hydroxide or sodium hydroxide may, for example, be used. The alkali compound may be preliminarily made in the state of an aqueous solution and added.

Then, this solution is filtrated, and the filtrate is recovered, and the extract is neutralized with an acid. As the acid, there is no particular limitation, and an organic acid or an inorganic acid may be used.

This neutralized solution is subjected to decoloring and desalting, and used as it is as a crude hemicellulose. Otherwise, it may be then concentrated, or further powdered by spray drying or freeze drying, and used as a crude hemicellulose.

Furthermore, it is possible to obtain hemicellulose with a desired purity by separating and removing proteins precipitated by neutralization in the neutralized solution by, for example, centrifugation, and if the case requires, treating the supernatant by dialysis, ion exchange resin treatment, ion exchange membrane treatment, ultrafiltration membrane treatment, alcohol purification, or a treatment with filtering aid, alone or in combination.

Further, the partially hydrolyzed product of hemicellulose used in the present invention may be prepared, for example, as mentioned below.

The cornhusk extract obtained by alkali treatment, is clarified and filtrated, and then subjected to pH adjustment, and reacted by adding xylanase at a temperature of 50 to 60°C. The added amount of xylanase is preferably from 0.0001 to 10 units per g of the solid content of the extract, and the reaction time is preferably from 3 to 96 hours.

The activity of xylanase is measured by using the hemicellulose obtained from the cornhusk by alkali extraction as a substrate, under the reaction conditions of pH 7 at 60°C, with the enzyme amount which will form a reducing sugar corresponding to 1 µmol of xylose per 1 minute being 1 unit.

Further, as the xylanase, ones of a liquefying type are preferred rather than the ones of a saccharifying type. Both ones derived from fungi and ones derived from bacteria may be used. However, bacterial xylanase is preferred by virtue of its high purity. As particularly preferred examples, an alkali xylanase having its optimum pH at the alkali side, described in JP-B-50-13357 may be mentioned.

Next, the enzyme is inactivated by heating or the like, and then the reaction solution is decolored and desalted, and concentrated, or further dried, whereby a partially hydrolyzed product of hemicellulose is obtained.

Further, the partially hydrolyzed product of hemicellulose may be obtained by preparing a solution containing from 3 to 30 % by mass of the hemicellulose purified as above, adjusting its pH if the case requires, and carrying out an enzymatic reaction in the same manner as above to inactivate the enzyme, and then separating it into a solid and a liquid by centrifugation or the like, followed by concentrating the supernatant and drying it.

The partially hydrolyzed product of hemicellulose prepared from cornhusk by the above manner is commercially available in the name of "celluace" (tradename, manufactured by Nihon Shokuhin Kako Co.,Ltd.,).

In the present invention, the mass average molecular weight of the used partially hydrolyzed product of hemicellulose prepared from cornhusk is preferably from 20,000 to 200,000, more preferably from 20,000 to 100,000, most preferably from 20,000 to 40,000. With respect to the partially hydrolyzed product of hemicellulose having such a mass average molecular weight, the viscosity of a 5 mass % aqueous solution is as low as from 3 to 20 cps (by a B model viscometer, 60 rpm, 25 °C), and when it is added to foodstuffs and beverages and the like, it functions to preserve the taste and flavor of them in good conditions. If the mass average molecular weight of the partially hydrolyzed product of hemicellulose exceeds 200,000, the viscosity tends to be too high and it is hardly added to the starting materials or the like of food and beverages, and if it is less than 20, 000, the physiological activity as dietary fibers tends to be impaired, such being undesirable.

The partially hydrolyzed hemicellulose is used as a main component of the immuno-active compositions, and may be commercialized as, for example, an aqueous solution, a concentrate or a dried powder containing the hemicellulose and/or its partially hydrolyzed product. In addition to the partially hydrolyzed hemicellulose starch, protein, and a small amount of lignin, cellulose, ashes, or the like may be contained.

Since the immuno-active agent manufactured according to the present invention can be easily dissolved in water even if it is in a dried powder state, it can be utilized as health food and beverages and medicines as it is. Further, by adding it in a small amount to food and beverages, it is possible to impart immuno-active effects without impairing the flavor and taste of the food and beverages.

When the immuno-active agent is added to food and beverages, the added amount thereof by which the immuno-active effect can be expected without impairing the taste of the food and beverages, is preferably from 0.1 to 10 % by mass. Further, the intake of the immuno-active agent of the present invention is preferably from 1 to 10 g/day, more preferably from 3 to 5 g/day, in order to develop the immnological activity action.

Hereinafter, the present invention will be described in further detail with reference to examples.

### Example

1,000 parts by mass of water and 1 part by mass of calcium hydroxide were added to 100 parts by mass of cornhusk, and heated at 85 °C for 3 hours. This reaction solution was cooled to 60°C, °C, and then sulfuric acid was added thereto to adjust the pH to 7.

Then, an alkali xylanase prepared by the method as described in JP-B-50-13357 ("Celluzyme", tradename, manufactured by Nihon Shokuhin Kako Co.,Ltd.,) was added in an amount of 0.01 unit per g of the solid content of this reaction solution, and reacted at 60 °C for 48 hours. After the completion of enzymatic reaction, this solution was heated at 90°C for 30 minutes to deactivate the enzyme, and then filtrated, and the filtrate was recovered, and further clarified and filtrated, decolored and desalted for purification, followed by spray drying with a spray dryer to obtain a powder of a partially hydrolyzed product of hemicellulose derived from cornhusk.

### Test Example 1

12 female mice of 4 weeks old were preliminarily reared for 7 days, and then separated into two groups (each group consists of 6 mice). To a test group, a solution of the partially hydrolyzed product of hemicellulose derived from cornhusk produced in the above example dissolved in distilled water, was orally administered with a gastric tube successively for 28 days (50 mg/kg a dose). Further, to a control group, distilled water was administered in the same manner as above. During the test period, the feed and water were freely ingested.

From 9:00 to 10:00 AM every day, the conditions were observed and the body weight and the like were measured. The change of the body weight, the feed consumption and the water consumption during the test period are indicated in Tables 1 and 2.

**Table 1**

| | | 1st day | 14th day | 28th day |
|---|---|---|---|---|
| Body weight (g) | Control group (n=6) | 14.62±1.05 | 21.85±0.66 | 23.93±0.36 |
| | Test group (n=6) | 14.28±1.12 | 21.92±0.92 | 23.68±0.98 |

**Table 2**

| | | 7th day | 14th day | 21st day | 28th day |
|---|---|---|---|---|---|
| Feed consumption (g/mouse) | Control | 3.10 | 3.50 | 3.70 | 4.00 |
| | Test | 3.20 | 3.60 | 3.70 | 3.90 |
| Water consumption (ml/mouse) | Control | 3.85 | 4.22 | 4.53 | 4.67 |
| | Test | 3.67 | 4.50 | 4.55 | 4.83 |
| *The values are average values (n=6). | | | | | |

From the results of Tables 1 and 2, it is evident that the body weight and the like were not influenced by the ingestion of the partially hydrolyzed product of hemicellulose derived from cornhusk. During the test period, general conditions of the mice were good, and no death or threatened killing was observed.

After the 28 days test, the abdominal region of each tested mouse was operated under anesthesia by nenbutal, the blood was collected from the abdominal portion of vena cava, and the spleen was enucleated. By the autopsy at the time of anatomy, no change attributable to the administration of the partially hydrolyzed product of hemicellulose derived from cornhusk was confirmed.

From the enucleated spleen, spleen cells were adjusted by a conventional manner, and the number of spleen cells was adjusted by a 10(v/v)% FCS-added RPMI-1640 culture media, and the cells were poured into a 24-well plate so that these would be 2 x 10⁶/ml/well.

Then, 100 µl of ConA (50 µg/ml) was added thereto to adjust the final concentration to be 5 µg/ml, and it was cultured in a 5% CO₂ incubator at 37 °C for 24 hours.

After the incubation, the suspension of cells of each well was filtrated with a 0.45 µm syringe filter, and the filtrate was used as samples for measurement of IL-2, INF-γ and IL-4.

The measurement of concentration of each cytokine was made by a plate leader system, using a Cytoscreeen kit of BIOSOURCE Co.

Each measured value was represented by "average value ± standard deviation". With respect to the examination of significant difference, the presence and absence of significant difference were judged by a Student's t-test with the risks of p<0.05 and p<0.01. The results are indicated in Table 3.

**Table 3**

| | | IL-2 | INF-γ | IL-4 |
|---|---|---|---|---|
| Concentration (pg/ml) | Control | 52.77±8.25 | 2.75±1.42 | 3.75±1.00 |
| | Test | 130.25±30.24** | 6.97±2.78** | 4.68±1.43 |

| | | | | |
|---|---|---|---|---|
| Average value± standard deviation: *p<0.05, **p<0.01 indicate significant differences from the control group. | | | | |

As is evident from Table 3, in the group to which the partially hydrolyzed product of hemicellulose derived from cornhusk was administered, apparent increase-accelerating action of the cytokine (IL-2, INF-γ and IL-4)-producing ability by the ConA stimulation to the spleen cells was confirmed. With respect to the IL-2 and INF-γ, significant difference (p<0.01) from the control group was confirmed.

From these results, it was confirmed that the ingestion of the partially hydrolyzed product of hemicellulose derived from cornhusk does not cause reactions such as antigenicity in organism at the time of usual life, and shows an action of accelerating the immune function sufficiently at the time of infection or stress.

### Industrial Applicability

As described above, according to the present invention, it is possible to provide an immuno-active agent derived from cornhusk, which is safe and can activate the immunity by orally ingesting it.

By ingesting the immuno-active agent, it is possible to enhance the immunities of persons whose immunities are deteriorating and who are susceptible to various diseases.

Further, the immuno-active agent can be ingested safely for a long period of time as medicines, food and beverages, or additives thereof. Since the partially hydrolyzed product of hemicellulose has a low viscosity, it is possible to preserve the taste and flavor of food and beverages in good conditions when it is added to them.

## Claims

1. Use of a partially hydrolyzed hemicellulose obtained from cornhusk and hydrolyzed with xylanase, wherein the hemicellulose is a hemicellulose obtained by removing starch and protein from the cornhusk and subjecting a residue to extraction with an alkali at a temperature of 80 to 140°C, in the manufacture of a composition for use in enhancing an immune response.

2. Use according to Claim 1, wherein the partially hydrolyzed product of hemicellulose has a mass average molecular weight of from 20,000 to 200,000.

3. Use according to Claim 1 or Claim 2, wherein said composition is to be administered orally.

4. Use according to Claim 3, wherein said composition accelerates production of a cytokine.

## Patentansprüche

1. Verwendung einer teilweise hydrolysierten Hemicellulose, die aus Maishülsen gewonnen und mit Xylanase hydrolysiert ist, wobei es sich bei der Hemicellulose um eine Hemicellulose handelt, die man erhält, indem man aus der Maishülse Stärke und Protein entfernt und den Rückstand einer Extraktion mit Alkali bei einer Temperatur von 80 bis 140 °C unterzieht, zur Herstellung eines Mittels zur Verstärkung einer Immunantwort.

2. Verwendung nach Anspruch 1, wobei das teilweise hydrolysierte Hemicelluloseprodukt ein massenmittleres Molekulargewicht von 20.000 bis 200.000 aufweist.

3. Verwendung nach Anspruch 1 oder 2, wobei das Mittel zur oralen Verabreichung bestimmt ist.

4. Verwendung nach Anspruch 3, wobei das Mittel die Produktion eines Cytokins beschleunigt.

## Revendications

1. Utilisation d'une hémicellulose partiellement hydrolysée obtenue à partir de spathe de maïs et hydrolysée avec de la xylanase, dans laquelle l'hémicellulose est une hémicellulose obtenue en retirant l'amidon et la protéine de la spathe de maïs et en soumettant un résidu à une extraction avec une base à une température de 80°C à 140°C, dans la fabrication d'une composition destinée à une utilisation pour accroître une réponse immune.

2. Utilisation selon la revendication 1, dans laquelle le produit partiellement hydrolysé d'hémicellullose possède une masse moléculaire moyenne en masse allant de 20 000 à 200 000.

3. Utilisation selon la revendication 1 ou la revendication 2, dans laquelle on doit administrer ladite composition par voie orale.

4. Utilisation selon la revendication 3, dans laquelle ladite composition accélère la production d'une cytokine.
